# EUROPÄISCHE PATENTANMELDUNG

(11) **EP 4 365 163 A1**
(43) Veröffentlichungstag der Anmeldung: **08.05.2024**
(21) Anmeldenummer: 22205113.8
(22) Anmeldetag: 02.11.2022
(51) Int. Cl.: C07C 209/26, C07C 211/50, C08K 3/04

(54) **VERFAHREN ZUR HERSTELLUNG VON N,N`-DIALKYL-P-PHENYLENDIAMIN**

(71) Anmelder: LANXESS Deutschland GmbH, 50569 Köln (DE)
(72) Erfinder: BÜHRLE, Miriam, 40670 Meerbusch Osterath (DE); GRAF, Holger, 42781 Haan (DE); WIEDEMEIER-JARAD, Melanie, 41540 Dormagen (DE); STUCKE, Nadja, 25462 Rellingen (DE); MINDACH, Olaf, 51379 Leverkusen (DE)

(57) **Zusammenfassung**

Die vorliegende Erfindung betrifft ein neues Verfahren zur Herstellung von *N*,*N*'-Dialkyl-*p-*phenylendiamin der Formel (I) sowie die Verwendung des *N*,*N*'-Dicyclohexyl-*p-*phenylendiamins in einer Reinheit von größer 95,0 Gew.-% als Alterungsschutzmittel in Kautschukmischungen, -vulkanisaten und daraus erhältlichen Formkörpern, insbesondere Reifen.

## Beschreibung

Die vorliegende Erfindung betrifft ein neues Verfahren zur Herstellung von *N,N*'-Dialkyl-*p-*phenylendiamin der Formel (I) sowie die Verwendung des *N,N*'-Dicyclohexyl-*p-*phenylendiamins in einer Reinheit von größer 95,0 Gew.-% als Alterungsschutzmittel in Kautschukmischungen, -vulkanisaten und daraus erhältlichen Formkörpern, insbesondere Reifen.

*N,N*'-Dicyclohexyl-p-phenylendiamin ist hervorragend geeignet, Vulkanisate auf Basis von Naturkautschuken oder Synthesekautschuken gegen Alterungsprozesse, bedingt durch die Einwirkung von Sauerstoff und Ozon, zu schützen. Es migriert an die Oberfläche des Vulkanisats und schützt dort den Kautschuk vor den zuvor genannten Einwirkungen.

CN1370768A beschreibt ein Verfahren zur Herstellung von *N,N*'-Di-sec-alkyl-*p-*phenylendiamin. p-Nitroanilin oder *p*-Phenylendiamin werden mit aliphatischen C₃-C₁₂ Ketonen in Gegenwart von CuO, Cr₂O₃ und BaO umgesetzt.

CN1947837A beschreibt ein Verfahren zur Herstellung von *N,N*'-Dialkyl-*p*-phenylendiamin. Als Katalysator wird ein Gemisch aus drei Komponenten gewählt: M1 (Ni, Co oder Fe), M2 (Re, Pd und/oder Ru) sowie M3 (Li, Ma, K, Rb, Ca, Mg, Sr oder Ba).

Nachteilig an beiden zuvor genannten Verfahren ist, dass mehrere Katalysatoren eingesetzt werden, die teils teuer und umweltbedenklich sind.

In CN105061214A wird ein Verfahren zur Herstellung von *N,N*'-Di-sec-butyl-*p-*phenylendiamin beschrieben, ausgehend von einer reduktiven Alkylierung von *p*-Nitroanilin und eines Ketons in Anwesenheit eines Platin-Katalysators sowie Aktivkohle. In diesem Verfahren wird nach Zugabe von p-Nitroanilin und Keton, hier Butanon, Aktivkohle zugegeben, um mögliche Verunreinigungen herauszufiltern. Erst nach Abtrennung der Aktivkohle durch Filtration wird der Katalysator zugegeben. Es wird eine Umsatzrate von 98.7% beschrieben, jedoch ist nicht beschrieben, in welcher Reinheit das Produkt letztlich vorliegt. Auch ist in dem beschriebenen Verfahren der Einsatz von Aktivkohle zur Reinigung der Ausgangsstoffe erforderlich. Ein Einsatz von Aktivkohle birgt jedoch einige Nachteile: Die Aktivkohle muss in einem weiteren Reaktionsschritt aus dem Reaktionsgemisch entfernt werden, was einen Mehraufwand sowie zusätzliche Reaktionsdauer erfordert. Darüber hinaus birgt Aktivkohle die Gefahr, Verunreinigungen im Reaktionsgemisch zu hinterlassen sowie beispielsweise Rohrleitungen und Filter zu verstopfen. Auch die Handhabung von Aktivkohlepulver ist problematisch, da der entstehende Staub einen erhöhten Aufwand aufgrund erhöhter Sicherheitsmaßnahmen erfordert.

In US4140718A wird ein Verfahren zur Herstellung von *N,N'*-Dialkyl-*p*-phenylendiaminen wie auch *N,N*'-Dicyclohexyl-p-phenylendiamin beschrieben, ausgehend von einer Umsetzung von *p*-Nitroanilin und eines Ketons in Anwesenheit von Wasserstoff und einem Hydrierkatalysator. Darin wird beschrieben, dass sich p-Nitroanilin schlecht in organischen Lösungsmitteln löst und es bei Verwendung von Ketonen mit geringem Molekulargewicht, wozu auch Cyclohexanon zählt, als Alkylierungsmittel bei Verwendung im Überschuss zum Ersatz beider Amin-Wasserstoffatome des aromatische Amins kommt. Es werden verschiedene Ether als Lösungsmittel eingesetzt. Die Reinheiten der Produkte sind mit 93 - 97% beschrieben. Allerdings werden die mono- und trialkylierten Nebenprodukte in Summe in 3 - 7 % erhalten, einzeln in jeweils mindestens 1%. Diese Nebenprodukte sind sehr unerwünscht, da sie die Migration des *N,N'*-Dialkyl-*p*-phenylendiamins, bevorzugt des *N,N'*-Dicyclohexyl-*p*-phenylendiamins, an die Oberfläche des Vulkanisats stören und damit im Fall von *N,N*'-Dicyclohexyl-p-phenylendiamin seine Wirksamkeit einschränken. So bildet das monoalkylierte Nebenprodukt aufgrund seiner freien Amin-Gruppe Wasserstoffbrückenbindungen mit anderen Molekülen, und das trialkylierte Nebenprodukt ist sterisch sehr anspruchsvoll und sperrig - beides Faktoren, die die oben beschriebene Migration behindern. Darüber hinaus wird in diesem Verfahren als Katalysator Platin aufgezogen auf Aluminium eingesetzt. Nachteilig daran ist, dass die Herstellung von Aluminium oft teuer und umweltschädlich ist. Auch ist die Hydrierzeit mit 4 - 5,2 Stunden relativ lang.

Es bestand daher Bedarf an einem Verfahren zur Herstellung von *N,N'*-Dialkyl-*p-*phenylendiamin, bevorzugt von *N,N'*-Dicycloalkyl-*p*-phenylendiamin, besonders bevorzugt von *N,N'*-Dicyclohexyl-*p*-phenylendiamin, das die vorgenannten Nachteile des Standes der Technik überwindet.

Aufgabe der vorliegenden Erfindung war es, ein Verfahren zur Herstellung von *N,N'*-Dialkyl-*p*-phenylendiamin, bevorzugt von *N,N'*-Dicycloalkyl-*p*-phenylendiamin, besonders bevorzugt von *N,N'*-Dicyclohexyl-*p*-phenylendiamin, bereit zu stellen, das die Nachteile des Standes der Technik überwindet, und *N,N'*-Dialkyl-*p*-phenylendiamin, bevorzugt *N,N'-*Dicycloalkyl-p-phenylendiamin, besonders bevorzugt *N,N'*-Dicyclohexyl-*p*-phenylendiamin, in hoher Reinheit und mit nur geringen Mengen der mono- und trialkylierten Nebenprodukte (im Rahmen dieser Erfindung bevorzugt *N*-Alkyl-*p*-phenylendiamin sowie *N,N,N'*-Trialkyl-*p-*phenylendiamin), herstellbar macht.

Die Reinheit der erhaltenen Produkte wird im Rahmen der vorliegenden Erfindung bevorzugt mittels Gaschromatographie gemäß ASTM-D 4937 ermittelt. Sie entspricht einer Reinheit in Gew.-%.

Eine hohe Reinheit im Rahmen der vorliegenden Erfindung liegt vor, wenn im Fall der Ermittlung der Reinheit über die Gaschromatographie das nach dem erfindungsgemäßen Verfahren hergestellte Produkt eine Reinheit von größer 95,0 Gew.-%, bevorzugt größer/gleich 96,0 Gew.-%, meist bevorzugt größer als 97,0 Gew.-%, aufweist.

Eine geringe Menge der mono- und trialkylierten Nebenprodukte (im Rahmen dieser Erfindung bevorzugt *N*-Alkyl-*p*-phenylendiamin sowie *N,N,N'*-Trialkyl-*p*-phenylendiamin) beträgt im Rahmen der vorliegenden Erfindung in Summe beider Nebenprodukte kleiner als 3,0 Gew.-%, bevorzugt kleiner als 2,5 Gew.-%, besonders bevorzugt kleiner als 1,5 Gew.-%, meist bevorzugt kleiner als 1,0 Gew.-%. Eine geringe Menge *N*-Alkyl-*p-*phenylendiamin liegt vor, wenn dies zu weniger als 1,0 Gew.-%, bevorzugt weniger als 0,5 Gew.-%, besonders bevorzugt weniger als 0,3 Gew.-% im Produkt enthalten ist. Eine geringe Menge *N,N,N'*-Trialkyl-*p*-phenylendiamin liegt vor, wenn dies zu weniger als 3 Gew.-%, bevorzugt weniger als 1,5 Gew.-%, besonders bevorzugt weniger als 1,0 Gew.-% im Produkt enthalten ist.

Überraschenderweise wurde gefunden, dass die Umsetzung einer Verbindung der Formel (II) mit wenigstens einem Keton der Formel (III) und einem Lösungsmittel der Formel (IVa), (IVb) oder (IVc), oder Mischungen davon, in Gegenwart eines Hydrierkatalysators und Wasserstoff zu hohen Reinheiten der entsprechenden *N,N'*-Dialkyl-*p*-phenylendiamine der Formel (I) führt, bei Erhalt nur geringer Mengen an mono- und trialkylierten Nebenprodukten.

### Verfahren

Gegenstand der vorliegenden Erfindung ist daher ein Verfahren zur Herstellung von *N,N'-*Dialkyl-*p*-phenylendiamin der Formel (I) 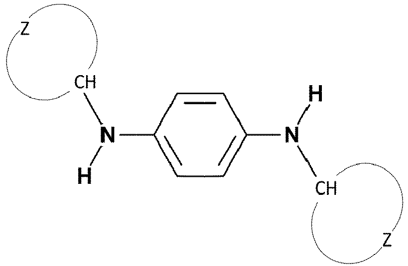
wobei eine Verbindung der Formel (II) 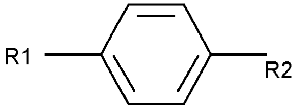 wobei R1 und R2 jeweils gleich oder verschieden sind und ausgewählt sind aus der Gruppe bestehend aus -NH₂ und -NO₂,
und wenigstens ein Keton der Formel (III) 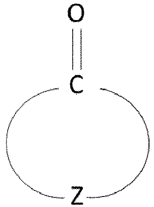 in Gegenwart eines Hydrierkatalysators und Wasserstoff umgesetzt werden,
wobei Z in den Formeln (I) und (III) gleich ist und
   a) für eine Alkylenkette, bevorzugt eine lineare Alkylenkette, steht, die mit dem an Z angrenzenden Kohlenstoffatom einen cyclischen Ring bildet, und wobei die Alkylenkette von Z bevorzugt C₄-C₆-Alkylen, besonders bevorzugt C₅-Alkylen, ist,
      oder
   b) für zwei Alkylreste, bevorzugt lineare Alkylreste, steht, welche jeweils mit dem an Z angrenzenden Kohlenstoffatom verbunden sind und gemeinsam mit diesem Kohlenstoffatom eine Alkylkette, bevorzugt eine lineare Alkylkette, bilden, und wobei die zwei Alkylreste von Z gemeinsam die Summenformel CₙH₂ₙ₊₂ bilden, bevorzugt mit n=4-6, besonders bevorzugt mit n=5,
dadurch gekennzeichnet, dass die Umsetzung in Gegenwart eines Lösungsmittels
der Formel (IVa) 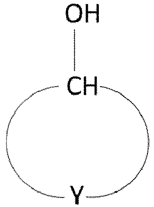
der Formel (IVb) 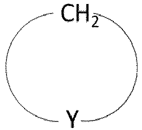
oder der Formel (IVc) 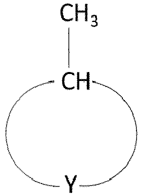
oder Mischungen davon,
erfolgt,
wobei Y in den Formeln (IVa), (IVb) und (IVc) gleich oder verschieden, bevorzugt gleich, ist und
   a) für eine Alkylenkette, bevorzugt eine lineare Alkylenkette, steht, die mit dem an Y angrenzenden Kohlenstoffatom einen cyclischen Ring bildet, und wobei die Alkylenkette von Y bevorzugt C₄-C₆-Alkylen, besonders bevorzugt C₅-Alkylen ist,
      oder
   b) für zwei Alkylreste, bevorzugt lineare Alkylreste, oder einen Alkylrest und ein Wasserstoffatom steht, welche jeweils mit dem an Y angrenzenden Kohlenstoffatom verbunden sind und gemeinsam mit diesem Kohlenstoffatom eine Alkylkette, bevorzugt eine lineare Alkylkette, bilden, und wobei die zwei Alkylreste oder der eine Alkylrest und das Wasserstoffatom gemeinsam die Summenformel CₙH₂ₙ₊₂ bilden, bevorzugt mit n=4-6, besonders bevorzugt mit n=5.

Der Begriff "Alkylen" steht in obigen Definitionen von Y und Z bevorzugt für CₙH₂ₙ.

In dem erfindungsgemäßen Verfahren können Y in den Formeln (IVa), (IVb) und (IVc) und Z in den Formeln (I) und (III) gleich oder verschieden sein. Bevorzugt sind Y und Z in den Formeln (I), (III), (IVa), (IVb) und (IVc) gleich.

In einer bevorzugten Ausführungsform des erfindungsgemäßen Verfahrens sind Z und Y in den Formeln (I), (III) und (IVa) gleich.

In einer weiteren bevorzugten Ausführungsform des erfindungsgemäßen Verfahrens sind Z und Y in den Formeln (I), (III) und (IVb) gleich.

In einer weiteren bevorzugten Ausführungsform des erfindungsgemäßen Verfahrens sind Z und Y in den Formeln (I), (III) und (IVc) gleich.

In einer weiteren Ausführungsform der vorliegenden Erfindung ist Z in den Formeln (I) und (III) gleich und verschieden von Y in den Formeln (IVb) bzw. (IVc).

Das erfindungsgemäße Verfahren ist ein Verfahren zur Herstellung von *N,N*'-Dialkyl-*p-*phenylendiamin der Formel (I).

Z kann in Formel (I) für zwei Alkylreste, bevorzugt lineare Alkylreste, stehen, welche jeweils mit dem an Z angrenzenden Kohlenstoffatom verbunden sind und gemeinsam mit diesem Kohlenstoffatom eine Alkylkette, bevorzugt eine lineare Alkylkette, bilden, und wobei die zwei Alkylreste von Z gemeinsam die Summenformel CₙH₂ₙ₊₂ bilden, bevorzugt mit n=4-6, besonders bevorzugt mit n=5.

Demnach kann das erfindungsgemäße Verfahren ein Verfahren zur Herstellung von *N,N'-*Alkyl-p-phenylendiamin sein, bevorzugt von *N,N'*-Pentyl-, *N,N'*-Hexyl-, *N,N'*- Heptyl-*p-*phenylendiamin, besonders bevorzugt von *N,N'*-Hexyl-*p*-phenylendiamin.

In einer bevorzugten Ausführungsform des erfindungsgemäßen Verfahrens steht Z in Formel (I) für eine Alkylenkette, bevorzugt eine lineare Alkylenkette, die mit dem an Z angrenzenden Kohlenstoffatom einen cyclischen Ring bildet, und wobei die Alkylenkette von Z bevorzugt C₄-C₆-Alkylen, besonders bevorzugt C₅-Alkylen ist.

Bevorzugt ist das erfindungsgemäße Verfahren demnach ein Verfahren zur Herstellung von *N,N'*-Cycloalkyl-*p*-phenylendiamin.

Besonders bevorzugt steht Z in Formel (I) für eine lineare Alkylenkette, die mit dem an Z angrenzenden Kohlenstoffatom einen cyclischen Ring bildet, und wobei die lineare Alkylenkette C₄-C₆-Alkylen, meist bevorzugt C₅-Alkylen ist.

Besonders bevorzugt ist das erfindungsgemäße Verfahren demnach ein Verfahren zur Herstellung von *N,N'*-Cyclopentyl-*p*-phenylendiamin, *N,N'*-Cyclohexyl-*p*-phenylendiamin und *N,N'*-Cycloheptyl-*p*-phenylendiamin, meist bevorzugt von *N,N'*-Cyclohexyl-*p-*phenylendiamin.

In dem erfindungsgemäßen Verfahren wird die Verbindung der Formel (II) eingesetzt, wobei R1 und R2 jeweils gleich oder verschieden sind und ausgewählt sind aus der Gruppe bestehend aus -NH₂ und -NO₂.

Bevorzugt sind R1 und R2 gleich und -NH₂, oder verschieden und -NH₂ und -NO₂, besonders bevorzugt verschieden und -NH₂ und -NO₂. Besonders bevorzugt ist die Verbindung der Formel (II) demnach para-Nitroanilin.

Es können beliebige Ketone der Formel (III) eingesetzt werden.

Y kann in dem wenigstens einen Keton der Formel (III) für zwei Alkylreste, bevorzugt lineare Alkylreste, stehen, welche jeweils mit dem an Z angrenzenden Kohlenstoffatom verbunden sind und gemeinsam mit diesem Kohlenstoffatom eine Alkylkette, bevorzugt eine lineare Alkylkette, bilden, und wobei die zwei Alkylreste von Z gemeinsam die Summenformel CₙH₂ₙ₊₂ bilden, bevorzugt mit n=4-6, besonders bevorzugt mit n=5.

Demnach kann als wenigstens ein Keton der Formel (III) ein Keton ausgewählt aus der Gruppe Pentanon, Hexanon und Heptanon eingesetzt werden, bevorzugt ausgewählt aus Pentan-2-on, Pentan-3-on, Hexan-2-on, Hexan-3-on, Heptan-2-on, Heptan-3-on und Heptan-4-on.

Gemäß obiger Bevorzugung von Y in Formel (III) ist das in Schritt a) eingesetzte wenigstens eine Keton der Formel (III) bevorzugt ein cyclisches Keton, besonders bevorzugt ausgewählt aus Cyclopentanon, Cyclohexanon und Cycloheptanon.

Meist bevorzugt ist das Keton der Formel (III) demnach Cyclohexanon.

Bevorzugt wird in dem erfindungsgemäßen Verfahrens ein Keton der Formel (III) eingesetzt.

Es können beliebige Lösungsmittel der Formeln (IVa), (IVb) oder (IVc) oder Mischungen davon eingesetzt werden.

Y kann in dem Lösungsmittel der Formel (IVa), (IVb) oder (IVc) für zwei Alkylreste, bevorzugt lineare Alkylreste, oder einen Alkylrest und ein Wasserstoffatom stehen, welche jeweils mit dem an Y angrenzenden Kohlenstoffatom verbunden sind und gemeinsam mit diesem Kohlenstoffatom eine Alkylkette, bevorzugt eine lineare Alkylkette, bilden, und wobei die zwei Alkylreste oder der eine Alkylrest und das Wasserstoffatom gemeinsam die Summenformel CₙH₂ₙ₊₂ bilden, bevorzugt mit n=4-6, besonders bevorzugt mit n=5.

Demnach kann als Lösungsmittel der Formeln (IVa), (IVb) oder (IVc) oder Mischungen davon ein Lösungsmittel bevorzugt ausgewählt aus der Gruppe Pentanol, Hexanol und Heptanol, oder Mischungen davon, eingesetzt werden, besonders bevorzugt Hexanol.

Bevorzugt steht Y in den Formeln (IVa), (IVb) oder (IVc) für eine Alkylenkette, bevorzugt eine lineare Alkylenkette, die mit dem an Y angrenzenden Kohlenstoffatom einen cyclischen Ring bildet, und wobei die Alkylenkette von Y bevorzugt C₄-C₆-Alkylen, besonders bevorzugt C₅-Alkylen ist.

Gemäß obiger Bevorzugung von Y in Formel (IVa) ist das eingesetzte Lösungsmittel der Formel (IVa) bevorzugt ausgewählt aus Cyclopentanol, Cyclohexanol und Cycloheptanol, oder Mischungen davon.

Meist bevorzugt ist das Lösungsmittel der Formel (IVa) Cyclohexanol.

Gemäß obiger Bevorzugung von Y in Formel (IVb) ist das eingesetzte Lösungsmittel der Formel (IVb) bevorzugt ausgewählt aus Cyclopentan, Cyclohexan und Cycloheptan, oder Mischungen davon.

Meist bevorzugt ist das Lösungsmittel der Formel (IVb) Cyclohexan.

Gemäß obiger Bevorzugung von Y in Formel (IVc) ist das eingesetzte Lösungsmittel der Formel (IVc) bevorzugt ausgewählt aus Methylcyclopentan, Methylcyclohexan und Methylcycloheptan, oder Mischungen davon.

Meist bevorzugt ist das Lösungsmittel der Formel (IVc) Methylcyclohexan.

Bevorzugt ist das Lösungsmittel der Formel (IV) ein Lösungsmittel der Formel (IVb) oder (IVc), oder Mischungen davon, besonders bevorzugt ausgewählt aus Cyclohexan und Methylcyclohexan.

Meist bevorzugt ist das Lösungsmittel der Formel (IV) Methylcyclohexan.

Ether, bevorzugt ausgewählt aus der Gruppe bestehend aus Monoethern aus Gylkolen, Diethern aus Gylkolen und cyclischen Diethern, sind in dem erfindungsgemäßen Verfahren bevorzugt in einem molaren Verhältnis zu der Verbindung der Formel (II) von weniger als 0,2 : 1,0, besonders bevorzugt von weniger als 0,05 :1, ganz besonders bevorzugt von weniger als 0,01 : 1,0 zugegen. Meist bevorzugt sind in dem erfindungsgemäßen Verfahren keine Ether, bevorzugt ausgewählt aus der Gruppe bestehend aus Monoethern aus Gylkolen, Diethern aus Gylkolen und cyclischen Diethern, zugegen.

Die zuvor genannten Ausführungen und Bevorzugungen von Z in den Formeln (I) und (III) und Y in den Formeln (IVa), (IVb) und (IVc) gelten folglich nicht nur für die Umsetzung in dem erfindungsgemäßen Verfahren, sondern auch für die sich optional anschließende Aufarbeitung.

Der in dem erfindungsgemäßen Verfahren eingesetzte Hydrierkatalysator kann ein für Hydrierungen aus dem Stand der Technik bekannter und gängiger Katalysator sein.

Bevorzugt enthält der Hydrierkatalysator ein Metall ausgewählt aus der Gruppe bestehend aus Platin, Palladium und Nickel, besonders bevorzugt Platin.

Der Hydrierkatalysator kann auf einem festen Trägermaterial aufgezogen sein, beispielsweise Kohlenstoff, Aluminium, Bariumsulfat oder Calciumcarbonat. Bevorzugt ist der Hydrierkatalysator ein Katalysator auf Kohlenstoff.

Besonders bevorzugt ist der Hydrierkatalysator Platin auf Kohlenstoff (Pt/C).

Der Hydrierkatalysator kann eine beliebige Menge Platin enthalten. Bevorzugt enthält der Hydrierkatalysator 1 - 10 Gew.-% Platin, besonders bevorzugt 1,5 - 5 Gew.-% Platin, ganz besonders bevorzugt 2 - 4 Gew.-% Platin, ganz besonders bevorzugt 2,5 - 3,5 Gew.-% Platin, meist bevorzugt 3 Gew.-% Platin.

Der Einsatz des Hydrierkatalysators Platin auf Kohlenstoff löst die weitere Aufgabe, ein umweltfreundlicheres Verfahren bereitzustellen, verglichen mit dem in US4140718A offenbarten, welches Platin auf Aluminium als Hydrierkatalysator einsetzt.

Der Hydrierkatalysator kann Wasser enthalten oder nicht enthalten. Bevorzugt enthält der Katalysator Wasser, besonders bevorzugt 30 - 70 Gew.-%, ganz besonders bevorzugt 50 - 70 Gew.-%, meist bevorzugt 60 - 65 Gew.-%.

Der Hydrierkatalysator kann vor dem Einsatz in dem erfindungsgemäßen Verfahren entwässert werden. Die Entwässerung kann auf verschiedene bekannte Weisen erfolgen, beispielsweise über eine azeotrope Destillation. Bevorzugt erfolgt die Entwässerung über eine azeotrope Destillation. Dafür können verschiedene Lösungsmittel und Lösungsmittelgemische eingesetzt werden.

Die zur Umsetzung in dem erfindungsgemäßen Verfahren eingesetzten Komponenten können in verschiedenen Verhältnissen zueinander eingesetzt werden.

Bevorzugt wird das Keton der Formel (III) im molaren Verhältnis 1,2 : 1,0 bis 5,0 :1,0, besonders bevorzugt 1,5 : 1,0 bis 3,0 : 1,0, ganz besonders bevorzugt 1,8 : 1,0 bis 2,5 : 1,0, zu der Verbindung der Formel (II) eingesetzt.

Bevorzugt wird das Lösungsmittel der Komponente (IV) im molaren Verhältnis 1,5 : 1,0 bis 10,0 :1,0, besonders bevorzugt 2,0 : 1,0 bis 8,0 : 1,0, ganz besonders bevorzugt 3,0 : 1,0 bis 5,0 : 1,0, zu der Verbindung der Formel (II) eingesetzt.

Bevorzugt wird der Hydrierkatalysator im Gewichtsverhältnis 1,0 : 100,0 bis 6,0 :100,0, besonders bevorzugt 2,0 : 100,0 bis 6,0 : 100,0, ganz besonders bevorzugt 2,0 : 1,0 bis 4,0 : 100,0, zu der Verbindung der Formel (II) eingesetzt.

Die für die Umsetzung eingesetzten Komponenten können ohne eine vorherige Reinigung eingesetzt werden oder vor der Umsetzung gereinigt werden. Bevorzugt werden die für die Umsetzung eingesetzten Komponenten ohne eine vorherige Reinigung eingesetzt. Besonders bevorzugt werden die für die Umsetzung eingesetzten Komponenten in Reinheiten von ≥ 90%, ganz besonders bevorzugt ≥ 95%, ganz besonders bevorzugt ≥ 98%, meist bevorzugt ≥ 99% eingesetzt.

Die für die Umsetzung eingesetzten Komponenten können in beliebiger Reihenfolge vorgelegt werden.

Bevorzugt werden zunächst die Verbindungen der Formeln (II), (III) und (IV) vorgelegt.

Bevorzugt werden die eingesetzten Komponenten des erfindungsgemäßen Verfahrens vor der Umsetzung einer Schutzgasatmosphäre ausgesetzt, wie beispielsweise einer Argon- oder Stickstoffatmosphäre, bevorzugt einer Stickstoffatmosphäre.

Die Umsetzung erfolgt bevorzugt bei einer Temperatur von 100 - 170 °C, besonders bevorzugt bei 90 - 150 °C, ganz besonders bevorzugt bei 95 - 140 °C, meist bevorzugt bei 125 - 135 °C.

Die Umsetzung erfolgt bevorzugt bei einem Wasserstoffdruck von 5 - 30 bar, besonders bevorzugt von 10 - 20 bar, ganz besonders bevorzugt von 13 - 17bar.

Die Umsetzung erfolgt bevorzugt solange, bis keine weitere Aufnahme von Wasserstoff mehr detektiert werden konnte. Die Detektion der Wasserstoffaufnahme erfolgt bevorzugt durch Kontrolle der Druckänderung an einem Manometer. Besonders bevorzugt erfolgt die Detektion in Abständen von weniger als 60 min, ganz besonders bevorzugt von weniger als 45 min, meist bevorzugt von weniger als 35 min.

Die Umsetzungsdauer beträgt bevorzugt 50 min bis 400 min, besonders bevorzugt 80 min bis 300 min, ganz besonders bevorzugt 100 bis 270 min, meist bevorzugt 100 bis 200 min.

Nach der erfolgten Umsetzung kann das Reaktionsgemisch noch weiter gerührt werden. Bevorzugt erfolgt ein solches Nachrühren für 10 min bis 200 min, besonders bevorzugt für 15 min bis 100 min, ganz besonders bevorzugt für 25 min bis 70 min.

In einer bevorzugten Ausführungsform des erfindungsgemäßen Verfahrens ist das Verfahren ein Verfahren zur Herstellung von *N,N'*-Dicycloalkyl-*p*-phenylendiamin der Formel (I), wobei Y und Z in den Formeln (I), (III) und (IVa), (IVb) und (IVc) gleich sind und für eine lineare Alkylenkette stehen, welche bevorzugt C₅-Alkylen ist.

Besonders bevorzugt ist das Lösungsmittel der Formel (IV) in der zuletzt genannten bevorzugten Ausführungsform ausgewählt aus einem Lösungsmittel der Formeln (IVa) und (IVb).

Diese besonders bevorzugte Ausführungsform löst die weitere Aufgabe, ein effizienteres Verfahren bereitzustellen, verglichen mit dem in US4140718A offenbarten, welches Hydrierzeiten von 4 - 5,2 Stunden benötigt.

Bevorzugt ist das erfindungsgemäße Verfahren ein Verfahren zur Herstellung von *N,N'-*Dialkyl-p-phenylendiamin der Formel (I) in einer Reinheit von größer 95,0 Gew.-%, besonders bevorzugt größer/gleich 96,0 Gew.-%, meist bevorzugt größer als 97,0 Gew.-%, bevorzugt ermittelt mittels Gaschromatographie gemäß ASTM-D 4937.

Im Rahmen dieser Anmeldung ist der Ausdruck "Herstellung von *N,N'*-Dialkyl-*p-*phenylendiamin der Formel (I) in einer Reinheit von größer 95,0 Gew.-%," gleichbedeutend mit "Herstellung einer Zusammensetzung enthaltend *N,N'*-Dialkyl-*p*-phenylendiamin der Formel (I) zu größer 95,0 Gew.-%". Entsprechendes gilt für analoge Textstellen in dieser Anmeldung wie auch im Abschnitt "Verwendung".

Bevorzugt ist das erfindungsgemäße Verfahren ein Verfahren zur Herstellung von *N,N'-*Dialkyl-p-phenylendiamin der Formel (I), welches die Verbindungen *N*-Alkyl-*p-*phenylendiamin sowie *N,N,N'*-Trialkyl-*p*-phenylendiamin in Summe kleiner als 3,0 Gew.-%, bevorzugt kleiner als 2,5 Gew.-%, besonders bevorzugt kleiner als 1,5 Gew.-%, meist bevorzugt kleiner als 1,0 Gew.-%, aufweist, bevorzugt ermittelt mittels Gaschromatographie gemäß ASTM-D 4937.

### Aufarbeitung

Nach der Umsetzung kann die erhaltene Reaktionsmischung auf verschiedene Weisen aufgearbeitet werden, die dem Fachmann bekannt sind.

In einer bevorzugten Ausführungsform des erfindungsgemäßen Verfahrens schließt sich der Umsetzung die Entfernung des Hydrierkatalysators aus der Reaktionsmischung erhalten nach der Umsetzung an.

Die Entfernung kann auf verschiedene, dem Fachmann bekannte Weisen erfolgen, wie beispielsweise Filtration, bevorzugt über eine Fritte oder Drucknutsche, oder Absaugen. Bevorzugt erfolgt die Entfernung durch Filtration der Reaktionsmischung erhalten nach der Umsetzung, besonders bevorzugt durch Filtration der heißen Reaktionsmischung.

Nach Entfernung des Hydrierkatalysators aus der Reaktionsmischung kann das Lösungsmittel der Reaktionsmischung erhalten nach der Entfernung des Hydrierkatalysators auf verschiedene, dem Fachmann bekannte Weisen entfernt werden.

Bevorzugt erfolgt die Entfernung durch Destillation der Reaktionsmischung erhalten nach Entfernung des Hydrierkatalysators. Die Temperatur der Destillation ist abhängig von dem Siedepunkt des eingesetzten Lösungsmittels. Bevorzugt erfolgt die Destillation bei vermindertem Druck.

Das nach der Entfernung des Lösungsmittels wiedergewonnene Lösungsmittelgemisch kann für eine erneute Hydrierung eingesetzt werden.

In einer besonders bevorzugten Ausführungsform schließt sich der Umsetzung des erfindungsgemäßen Verfahrens die Entfernung des Hydrierkatalysators aus der Reaktionsmischung erhalten nach der Umsetzung an sowie die Entfernung des Lösungsmittels aus der Reaktionsmischung erhalten nach Entfernung des Hydrierkatalysators. Die Bestimmung der Reinheit des *N,N'*-Dialkyl-*p*-phenylendiamins der Formel (I) sowie die Bestimmung der Anteile an *N*-Alkyl-*p*-phenylendiamin sowie *N,N,N'-*Trialkyl-p-phenylendiamin, bevorzugt ermittelt mittels Gaschromatographie gemäß ASTM-D 4937, erfolgt bevorzugt nach Entfernung des Hydrierkatalysators und des Lösungsmittels.

Nach Entfernung des Lösungsmittels aus der Reaktionsmischung kann der erhaltene Destillationsrückstand auf verschiedene, dem Fachmann bekannte Weisen aufgearbeitet werden.

Bevorzugt wird der erhaltene Destillationsrückstand abgekühlt, wobei das *N,N'*-Dialkyl-*p-*phenylendiamins der Formel (I) auskristallisiert.

Besonders bevorzugt wird der erhaltene heiße Destillationsrückstand auf ein Kristallisierblech gegeben.

Optional kann das erhaltene *N,N'*-Dialkyl-*p*-phenylendiamin der Formel (I) im Anschluss getrocknet werden.

Die Trocknung kann auf verschiedene, dem Fachmann bekannte Weisen erfolgen. Bevorzugt erfolgt die Trocknung im Vakuum, besonders bevorzugt bei einem Druck von kleiner als 10 kPa, ganz besonders bevorzugt bei einem Druck kleiner als 5 kPa.

### Verwendung

Weiterer Gegenstand der vorliegenden Erfindung ist die Verwendung von *N,N'-*Dicyclohexyl-p-phenylendiamin in einer Reinheit von größer 95,0 Gew.-%, bevorzugt größer/gleich 96,0 Gew.-%, meist bevorzugt größer als 97,0 Gew.-%, als Alterungsschutzmittel in Kautschukmischungen, -vulkanisaten und daraus erhältlichen Formkörpern, insbesondere Reifen, wobei die Reinheit bevorzugt ermittelt ist mittels Gaschromatographie gemäß ASTM-D 4937.

Weiter bevorzugt weist das *N,N*'-Dicyclohexyl-p-phenylendiamin die Verbindungen *N-*Cyclohexyl-*p*-phenylendiamin und *N,N,N'*-Tricyclohexyl-*p*-phenylendiamin in Summe kleiner als 3,0 Gew.-%, bevorzugt kleiner als 2,5 Gew.-%, besonders bevorzugt kleiner als 1,5 Gew.-%, meist bevorzugt kleiner als 1,0 Gew.-%, auf.

Besonders bevorzugt weist das *N,N*'-Dicyclohexyl-p-phenylendiamin die Verbindung *N-*Cyclohexyl-*p*-phenylendiamin in weniger als 0,5 Gew.-%, ganz besonders bevorzugt in weniger als 0,3 Gew.-% auf.

Besonders bevorzugt weist das *N,N*'-Dicyclohexyl-p-phenylendiamin die Verbindung *N,N,N'*-Tricyclohexyl-*p*-phenylendiamin in weniger als 3 Gew.-%, ganz besonders bevorzugt in weniger als 1,5 Gew.-%, meist bevorzugt in weniger als 1,0 Gew.-% auf.

Die angeführten Vorzugsbereiche gelten für die Verwendung in Kautschukmischungen, - vulkanisaten und Formkörpern, insbesondere Reifen, analog.

Ebenso gelten die im Rahmen dieser Erfindung angeführten Beschreibungen und Vorzugsbereiche unabhängig davon, ob sie im Plural oder im Singular offenbart wurden.

Die Erfindung soll anhand der folgenden Beispiele erläutert werden, ohne diese jedoch darauf zu beschränken.

### Beispiele

### Verfahren zur Herstellung von N,N'-Dicyclohexyl-p-phenylendiamin

**Tabelle 1: Liste der Einsatzstoffe, Abkürzungen und Hersteller**

| **Handelsname** | **Erläuterung** | **Hersteller/ Vertrieb** |
|---|---|---|
| p-Nitroanilin | Rohstoff, Reinheit ≥ 99% | Sigma-Aldrich |
| Cyclohexanon | Rohstoff, Reinheit ≥ 99% | Sigma-Aldrich |
| Cyclohexanol | Lösemittel, Reinheit ≥ 99% | Sigma-Aldrich |
| Evonik Noblyst^{®} P2067 3% Pt/C | Hydrierkatalysator: Platin/Aktivkohle (3 Gew.-% Platin, 62,5% Gew.-% H₂O) | Evonik Industries |
| Cyclohexan | Lösemittel, Reinheit ≥ 99% | Sigma-Aldrich |
| Methylcyclohexan | Lösemittel, Reinheit ≥ 99% | Sigma-Aldrich |

**Tabelle 2: Einsatzmengen der Einsatzstoffe und Reaktionsparameter**

| | **Beispiel 1** | **Beispiel 2** | **Beispiel 3** |
|---|---|---|---|
| p-Nitroanilin | 69 g (0,5 mol) | 69 g (0,5 mol) | 69 g (0,5 mol) |
| Cyclohexanon | 101 g (1,03 mol) | 101 g (1,03 mol) | 103 g (1,05 mol) |
| Katalysator | 2 g | 2 g | 4 g |
| Methylcyclohexan | 188 g (1,91 mol) | | |
| Cyclohexan | | 188 g (2,23 mol) | |
| Cyclohexanol | | | 188 g (1,88 mol) |
| Druck [bar H₂] | 15 | 15 | 15 |
| Temperatur [°C] | 130 | 130 | 100 |
| Aufnahme in bar H₂ | 135 | 130 | 146 |
| Umsetzungsdauer [min] | 120 | 152 | 262 |
| Nachrühren [min] | 60 | 60 | 30 |

Herstellung von N,N'-Dicyclohexyl-p-phenylendiamin anhand der in Tabelle 2 angegebenen Einsatzmengen der Einsatzstoffe und Reaktionsparameter der Beispiele 1-3

Die folgende Durchführung erfolgte jeweils für die Beispiele 1-3.

### Umsetzung

Es wurden in einem 3 L-Alloy-Autoklaven (großer Blattrührer, 600 U/min) bei Raumtemperatur p-Nitroanilin, Cyclohexanon, der Katalysator und das jeweilige Lösungsmittel des jeweiligen Beispiels 1, 2 bzw. 3 vorgelegt.

Der Autoklav wurde verschlossen und mit Stickstoff gespült. Es wurde bei Raumtemperatur ein Wasserstoffdruck von 5 bar eingestellt und anschließend auf die jeweilige Temperatur der Beispiele 1, 2 bzw. 3 geheizt. Bei Erreichen der Temperatur wurde der Druck auf 15 bar H₂ gesteigert und es wurde bis zur Nullaufnahme hydriert (<1 bar H₂ Aufnahme in 30 min). Im Anschluss wurde noch 30 bzw. 60 min bei entsprechender Temperatur und Druck nachgerührt. Es wurde auf 75°C abkühlen gelassen und der Autoklave wurde entspannt und die Reaktionslösung entnommen.

### Aufarbeitung

Die Reaktionslösung erhalten nach der Umsetzung wurde heiß über eine Drucknutsche vom Katalysator filtriert.

Der wiedergewonnene Katalysator kann für eine erneute Hydrierung eingesetzt werden.

Die Reaktionslösung wurde bei 120°C/ 50 mbar von Leichtsiedern befreit. Das Destillat war zweiphasig. Die organische Phase bestand hauptsächlich aus dem Lösemittel (>99%) und kann für eine erneute Hydrierung eingesetzt werden.

Im Destillationssumpf erhielt man eine Schmelze, die heiß auf ein Kristallisierblech gegeben wurde. Nach Abkühlen wurde der auskristallisierte Feststoff abgeschlagen, zerkleinert und abgefüllt.

**Tabelle 3: Reinheiten*¹ der gemäß Beispiel 1, 2 und 3 hergestellten Produkte in [Gew.-%]**

| | **Beispiel 1** | **Beispiel 2** | **Beispiel 3** |
|---|---|---|---|
| N-Cyclohexyl-p-phenylendiamin | 0,23 | 0,07 | 0,15 |
| N,N'-Dicyclohexyl-p-phenylendiamin | 96,45 | 97,73 | 96,00 |
| N,N,N'-Tricyclohexyl-p-phenylendiamin | 1,04 | 0,80 | 2,60 |

| | | | |
|---|---|---|---|
| ^{*1}: gemessen mittels Gaschromatographie nach ASTM-D 4937 nach Entfernung des Hydrierkatalysators und des Lösungsmittels | | | |

### Ergebnisse

Wie aus Tabelle 3 ersichtlich ist, wurden *N,N'*-Dicyclohexyl-*p*-phenylendiamin in den Beispielen 1, 2 und 3 in einer hohen Reinheit von bis zu 97,73 Gew.-% hergestellt. Die unerwünschten Nebenprodukte *N*-Cyclohexyl-*p*-phenylendiamin sowie *N,N,N'-*Tricyclohexyl-*p*-phenylendiamin wurden nur in geringen Mengen erhalten, in Summe in jedem Beispiel unter 3 Gew.-%, in den Beispielen 1 und 2 sogar unter 1,5 Gew.-%, in Beispiel 2 sogar unter 1 Gew.-%. Das monoalkylierte Nebenprodukt *N*-Cyclohexyl-*p-*phenylendiamin ist sogar in allen Beispielen 1, 2 und 3 in weniger als 0,3 Gew.-% zugegen, das trialkylierte Nebenprodukt in Beispiel 2 sogar unter 1 Gew.-%.

## Patentansprüche

1. Verfahren zur Herstellung von *N,N'*-Dialkyl-*p*-phenylendiamin der Formel (I) 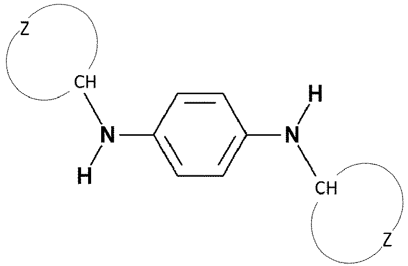
wobei eine Verbindung der Formel (II) 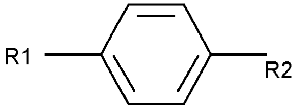
wobei R1 und R2 jeweils gleich oder verschieden sind und ausgewählt sind aus der Gruppe bestehend aus -NH₂ und -NO₂,
und wenigstens ein Keton der Formel (III) 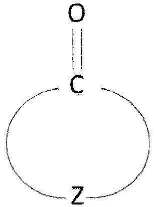
in Gegenwart eines Hydrierkatalysators und Wasserstoff umgesetzt werden,
wobei Z in den Formeln (I) und (III) gleich ist und
a) für eine Alkylenkette, bevorzugt eine lineare Alkylenkette, steht, die mit dem an Z angrenzenden Kohlenstoffatom einen cyclischen Ring bildet, und wobei die Alkylenkette von Z bevorzugt C₄-C₆-Alkylen, besonders bevorzugt C₅-Alkylen, ist,
oder
b) für zwei Alkylreste, bevorzugt lineare Alkylreste, steht, welche jeweils mit dem an Z angrenzenden Kohlenstoffatom verbunden sind und gemeinsam mit diesem Kohlenstoffatom eine Alkylkette, bevorzugt eine lineare Alkylkette, bilden, und wobei
die zwei Alkylreste von Z gemeinsam die Summenformel CₙH₂ₙ₊₂ bilden, bevorzugt mit n=4-6, besonders bevorzugt mit n=5,
**dadurch gekennzeichnet, dass** die Umsetzung in Gegenwart eines Lösungsmittels
der Formel (IVa) 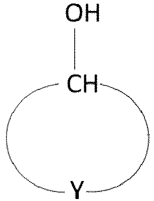
der Formel (IVb) 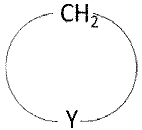
oder der Formel (IVc) 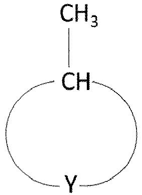
oder Mischungen davon,
erfolgt,
wobei Y in den Formeln (IVa), (IVb) und (IVc) gleich oder verschieden, bevorzugt gleich, ist und
a) für eine Alkylenkette, bevorzugt eine lineare Alkylenkette, steht, die mit dem an Y angrenzenden Kohlenstoffatom einen cyclischen Ring bildet, und wobei die Alkylenkette von Y bevorzugt C₄-C₆-Alkylen, besonders bevorzugt C₅-Alkylen ist,
oder
b) für zwei Alkylreste, bevorzugt lineare Alkylreste, oder einen Alkylrest und ein Wasserstoffatom steht, welche jeweils mit dem an Y angrenzenden Kohlenstoffatom verbunden sind und gemeinsam mit diesem Kohlenstoffatom eine Alkylkette, bevorzugt eine lineare Alkylkette, bilden, und wobei die zwei Alkylreste oder der eine Alkylrest und das Wasserstoffatom gemeinsam die Summenformel CₙH₂ₙ₊₂ bilden, bevorzugt mit n=4-6, besonders bevorzugt mit n=5.

2. Verfahren gemäß Anspruch 1, **dadurch gekennzeichnet, dass** Z in den Formeln (I) und (III) für eine Alkylenkette, bevorzugt eine lineare Alkylenkette steht, die mit dem an Z angrenzenden Kohlenstoffatom eine cyclischen Ring bildet, und wobei die Alkylenkette von Z bevorzugt C₄-C₆-Alkylen, besonders bevorzugt C₅-Alkylen, ist.

3. Verfahren gemäß einem der Ansprüche 1 oder 2, **dadurch gekennzeichnet, dass** Y in den Formeln (IVa), (IVb) und (IVc) gleich ist und für eine Alkylenkette, bevorzugt eine lineare Alkylenkette steht, die mit dem an Y angrenzenden Kohlenstoffatom eine cyclischen Ring bildet, und wobei die Alkylenkette von Y bevorzugt C₄-C₆-Alkylen, besonders bevorzugt C₅-Alkylen ist.

4. Verfahren gemäß einem de Ansprüche 1-3, **dadurch gekennzeichnet, dass** der Hydrierkatalysator 1 - 10 Gew.-% Platin, bevorzugt 1,5 - 5 Gew.-% Platin, besonders bevorzugt 2 - 4 Gew.-% Platin, ganz besonders bevorzugt 2,5 - 3,5 Gew.-% Platin, meist bevorzugt 3 Gew.-% Platin enthält.

5. Verfahren gemäß einem der Ansprüche 1-4, **dadurch gekennzeichnet, dass** das Lösungsmittel der Formel (IV) ausgewählt ist aus einem Lösungsmittel der Formeln (IVb) und (IVc), oder Mischungen davon, besonders bevorzugt ausgewählt aus Cyclohexan und Methylcyclohexan.

6. Verfahren gemäß einem der Ansprüche 1-5, **dadurch gekennzeichnet, dass** die Umsetzung bei einem Wasserstoffdruck von 5 - 30 bar erfolgt, bevorzugt von 10 - 20 bar, besonders bevorzugt von 13 - 17bar.

7. Verfahren gemäß einem der Ansprüche 1-6, **dadurch gekennzeichnet, dass** die Umsetzung bei einer Temperatur von 100 - 170 °C erfolgt, bevorzugt bei 90 - 150 °C, besonders bevorzugt bei 95 - 140 °C, ganz besonders bevorzugt bei 125 - 135 °C.

8. Verfahren gemäß einem der Ansprüche 1-7, **dadurch gekennzeichnet, dass** die Umsetzungsdauer 50 min bis 400 min beträgt, bevorzugt 80 min bis 300 min, besonders bevorzugt 100 bis 270 min, ganz besonders bevorzugt 100 bis 200 min.

9. Verfahren gemäß einem der Ansprüche 1-8, **dadurch gekennzeichnet, dass** das Verfahren ein Verfahren zur Herstellung von *N,N'*-Dialkyl-*p*-phenylendiamin der Formel (I) in einer Reinheit von größer 95,0 Gew.-% ist, bevorzugt größer/gleich 96,0 Gew.-%, besonders bevorzugt größer als 97,0 Gew.-%, bevorzugt ermittelt mittels Gaschromatographie gemäß ASTM-D 4937.

10. Verfahren gemäß einem der Ansprüche 1-9, **dadurch gekennzeichnet, dass** das Verfahren ein Verfahren zur Herstellung von *N,N'*-Dialkyl-*p*-phenylendiamin der Formel (I) ist, welches die Verbindungen *N*-Alkyl-*p*-phenylendiamin sowie *N,N,N'*-Trialkyl-*p-*phenylendiamin in Summe kleiner als 3,0 Gew.-%, bevorzugt kleiner als 2,5 Gew.-%, besonders bevorzugt kleiner als 1,5 Gew.-%, meist bevorzugt kleiner als 1,0 Gew.-%, aufweist, bevorzugt ermittelt mittels Gaschromatographie gemäß ASTM-D 4937.

11. Verfahren gemäß einem der Ansprüche 1-10, **dadurch gekennzeichnet, dass** das wenigstens eine Keton der Formel (III) im molaren Verhältnis 1,2 : 1,0 bis 5,0 :1,0, bevorzugt 1,5 : 1,0 bis 3,0 : 1,0, besonders bevorzugt 1,8 : 1,0 bis 2,5 : 1,0, zu der Verbindung der Formel (II) eingesetzt wird.

12. Verfahren gemäß einem der Ansprüche 1-11, **dadurch gekennzeichnet, dass** das Verfahren ein Verfahren zur Herstellung von *N,N'*-Dicycloalkyl-*p*-phenylendiamin der Formel (I) ist, wobei Y und Z in den Formeln (I), (III) und (IVa), (IVb) und (IVc) gleich sind und für eine lineare Alkylenkette stehen, welche bevorzugt C₅-Alkylen ist.

13. Verwendung von *N,N*'-Dicyclohexyl-p-phenylendiamin in einer Reinheit von größer 95,0 Gew.-%, bevorzugt größer/gleich 96,0 Gew.-%, meist bevorzugt größer als 97,0 Gew.-%, als Alterungsschutzmittel in Kautschukmischungen, -vulkanisaten und daraus erhältlichen Formkörpern, insbesondere Reifen, wobei die Reinheit bevorzugt ermittelt ist mittels Gaschromatographie gemäß ASTM-D 4937.

14. Verwendung gemäß Anspruch 13, **dadurch gekennzeichnet, dass** das *N,N'-*Dicyclohexyl-p-phenylendiamin die Verbindungen *N*-Cyclohexyl-*p*-phenylendiamin und *N,N,N'*-Tricyclohexyl-*p*-phenylendiamin in Summe kleiner als 3,0 Gew.-%, bevorzugt kleiner als 2,5 Gew.-%, besonders bevorzugt kleiner als 1,5 Gew.-%, meist bevorzugt kleiner als 1,0 Gew.-%, aufweist, wobei die Reinheit bevorzugt ermittelt ist mittels Gaschromatographie gemäß ASTM-D 4937.

15. Verfahren gemäß einem der Ansprüche 9 oder 10 oder Verwendung gemäß einem der Ansprüche 13 oder 14, **dadurch gekennzeichnet, dass** die Bestimmung der Reinheit des *N,N'*-Dialkyl-*p*-phenylendiamins der Formel (I) gemäß Anspruch 9 oder Anspruch 13 sowie die Bestimmung der Anteile an *N*-Alkyl-*p*-phenylendiamin sowie *N,N,N'-*Trialkyl-p-phenylendiamin gemäß Anspruch 10 oder Anspruch 14, bevorzugt ermittelt mittels Gaschromatographie gemäß ASTM-D 4937, nach Entfernung des Hydrierkatalysators und des Lösungsmittels erfolgt.
